# EUROPÄISCHE PATENTSCHRIFT

(11) **EP 1 878 379 B1**
(45) Veröffentlichungstag und Bekanntmachung des Hinweises auf die Patenterteilung: **16.10.2019**
(21) Anmeldenummer: 06014316.1
(22) Anmeldetag: 11.07.2006
(51) Int. Cl.: A61B 5/00, G01N 33/487, G01N 21/86

(54) **Testbandsystem, insbesondere für Blutzuckeranalyse**
Test tape system, in particular for blood sugar analysis
Système à bande de teste, en particulier pour l'analyse du sucre sanguin

(43) Veröffentlichungstag der Anmeldung: 16.01.2008
(73) Patentinhaber: F. Hoffmann-La Roche AG, 4070 Basel (CH); Roche Diabetes Care GmbH, 68305 Mannheim (DE)
(72) Erfinder: Sacherer, Klaus Dieter, 67281 Kirchheim (DE)
(74) Vertreter: Pfiz, Thomas

(56) Entgegenhaltungen:
- EP-A- 1 424 040
- WO-A-2005/047861
- DE-C- 639 177
- US-A- 6 040 191
- US-A1- 2002 188 224

## Beschreibung

Die Erfindung betrifft ein Testsystem zum Einsatz einer Testbandeinheit gemäß dem Oberbegriff des Patentanspruchs 1.

Für die Selbstdiagnose von Diabetikern werden bislang in der Praxis einzelne Teststreifen eingesetzt, die nach der Applikation einer geringen Probenmenge photometrisch untersucht werden, um den Glucosegehalt in der Probe (Blut bzw. Gewebeflüssigkeit) möglichst genau und zuverlässig zu bestimmen. Zur Verbesserung der Anwenderfreundlichkeit wurde bereits vorgeschlagen, eine Vielzahl von Tests auf einem Testband in Form einer Bandkassette bereitzustellen. Solche Bandkassetten sollen sich als Einwegteil in kompakte Handgeräte einsetzen lassen, um alle erforderlichen Untersuchungsschritte automatisch und schnell ausführen zu können. Hierbei ist zu beachten, dass die Verbrauchsteile ein Massenprodukt darstellen, an welches aufgrund der erforderlichen Zuverlässigkeit hohe Anforderungen gestellt werden.

In der WO 2005/006985 ist in diesem Zusammenhang eine bogenförmig gekrümmte Testbandführung vorgesehen, um einen Testbandabschnitt für einen vorderseitigen Flüssigkeitsauftrag zu exponieren und eine rückseitige optische Vermessung über eine auf diesen Bereich fokussierte reflektionsphotometrische Messeinheit. Bei einer solchen Anordnung mit parallelen Bogenschienen besteht jedoch bei einem dünnen flexiblen Testband die Gefahr, dass sich der zentrale Bandbereich unter Zug in den Freiraum zwischen den Schienen einwölbt, wodurch eine genaue optische Fokussierung erschwert wird. Es hat sich gezeigt, dass eine solche Bandverformung bereits bei geringen Krümmungsradien vor allem hinsichtlich einer kurzbrennweitigen Messoptik problematisch ist.

Um hier Abhilfe zu schaffen, wurde bereits vorgeschlagen, optische Elemente insbesondere in Form von Zylinderlinsen für eine Applikationsspitze einzusetzen, wobei die Linse im Strahlengang des Photometers das Messlicht bündelt. Hierbei ist jedoch wie bei einfachen optischen Fenstern zu beachten, dass für die erforderliche Messgenauigkeit hohe Qualitätsanforderungen gestellt werden, insbesondere hinsichtlich Transmission, Kratzfestigkeit, Temperaturbeständigkeit, Ausdehnungskoeffizient, optischer Güte und weiterer Materialparameter bzw. -fehler, wie sie insbesondere in der internationalen Norm ISO 10110 aufgeführt werden.

Ausgehend hiervon liegt der Erfindung die Aufgabe zugrunde, die im Stand der Technik vorgeschlagenen Testbandsysteme weiter zu verbessern und besondere Anwendungsvorteile zu erreichen, insbesondere eine hohe Messgenauigkeit bei geringer Beanspruchung des Testbands.

Zur Lösung dieser Aufgabe wird die im Patentanspruch 1 angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, ohne ein optisches Bauelement bzw. materielles Fenster im Bereich der Bandführung auszukommen. Es sollen also solche Optikelemente vermieden werden, in welchen Licht beim Durchtritt in Wechselwirkung mit dem optischen Material tritt. Dementsprechend wird gemäß der Erfindung vorgeschlagen, dass die Bandführung einen ebenen Auflagerahmen aufweist, welcher den Testbandabschnitt an der Messstelle plan aufspannt und dabei mit seinen Rahmenschenkeln eine von Optikelementen freigehaltene lichte, also nur luftgefüllte Messöffnung für eine bandrückseitige optische Messung umlaufend begrenzt, während an dem auf dem Auflagerahmen aufliegenden Testbandabschnitt unter freier Durchstrahlung der Messöffnung eine reflektometrische Messung von der Bandrückseite her erfolgt, wobei der Auflagerahmen zwei in Längsrichtung des Testbands nebeneinander verlaufende Rahmenschenkel aufweist, deren Außenrandabstand kleiner als die Breite des Testbands ist. Durch den überlappenden Testbandrand kann so eine Abschirmung gegen eine Gerätekontamination mit Probenflüssigkeit erreicht werden. Weiterhin kann eine gute Zielgenauigkeit und ausreichende Abstützung für den vorderseitigen Flüssigkeitsauftrag sichergestellt werden, während für die rückseitige optische Messung das Testband ohne merkliche Durchbiegung in einer eng definierten Messebene gehalten werden kann, wobei die lichte Messöffnung als einfacher optischer Zugang eine Lichteinstrahlung und einen Durchlass der reflektierenden Strahlung unter stets gleich bleibenden Bedingungen erlaubt. Hinzu kommt, dass keine speziellen optischen Bauteile wie Linsen, Filter oder materielle Fenster gefertigt werden müssen, und dass durch die Vermeidung von Bandeinschnürungen ein energieeffizienter und schonender Bandtransport möglich ist.

Vorteilhafterweise begrenzen die Rahmenschenkel des Auflagerahmens die Messöffnung rechteckförmig, wodurch auch ausreichend Raum für mehrere auf das Testband ausgerichtete Lichtbündel zur Verfügung steht.

Eine weitere Verbesserung wird dadurch erreicht, dass der Auflagerahmen zwei in Querrichtung des Testbands parallel verlaufende Rahmenschenkel aufweist, deren Länge zumindest der Breite des Testbands entspricht.

Auf diese Weise lässt sich im Umlenkbereich eine Bandeinschnürung vermeiden, so dass eine plane Rahmenüberspannung unterstützt wird.

Um die Bandführung weiter zu verbessern, ist es von Vorteil, wenn die das Testband in seiner Längsrichtung abstützenden Rahmenschenkel des Auflagerahmens streifenförmig abgeflacht sind. Günstig ist es auch, wenn die in Bandquerrichtung verlaufenden Rahmenschenkel an einer Umlenkkante für das Testband abgerundet sind.

In weiterer vorteilhafter Ausführung ist der Auflagerahmen durch die ebene Deckfläche eines vorzugsweise pyramidenstumpfförmig verjüngten Vorsprungs der Bandführung gebildet. Dadurch wird die Handhabung und Hygiene beim Flüssigkeitsauftrag zusätzlich verbessert.

Für die Applikation ist es auch vorteilhaft, wenn die Bandführung in Bandlängsrichtung an dem Auflagerahmen anschließende Umlenkschrägen aufweist, wobei die Umlenkschrägen mit der Ebene des Auflagerahmens einen spitzen Winkel einschließen.

Um das Testband gegen Querauslenkung sichern, kann die Bandführung vorteilhafterweise außerhalb des Bereichs des Auflagerahmens angeordnete Seitenbegrenzungen aufweisen, so dass auch eine exakte Zentrierung auf dem Auflagerahmen möglich ist.

Weiter ist es von Vorteil, wenn das Testband durch Antrieb der Aufwickelspule von der Vorratsspule abspulbar ist, wobei für die Planhaltung das Testband durch Rückhaltekräfte vorzugsweise von mehr als 1 N unter Zug gehalten sein sollte.

Für eine Massenfertigung ergeben sich Vorteile, wenn der Auflagerahmen an einem Formteil einstückig angeformt ist. Ein solches Formteil kann vorteilhafterweise als Spritzgussteil aus Polypropylen bestehen, wobei durch eine schwarze Einfärbung der Optikbereich gegen Einstreuung von Fremdlicht besser abgeschirmt wird.

Wenn der Probenauftrag und die Messung am selben Ort erfolgen, ist kein zusätzlicher Zwischentransport des mit Probe beaufschlagten Testbandabschnitts zu einer beabstandeten Messstelle erforderlich ist. In diesem Zusammenhang ist es vorteilhaft, wenn die Körperflüssigkeit auf die freie Bandvorderseite des auf dem Auflagerahmen abgestützten Testbandabschnitts applizierbar ist.

Um einen Geräteinsatz zu vereinfachen, ist es günstig, wenn eine von der Bandführung begrenzte Messkammer für den Einsatz einer Messeinheit ausgebildet ist, wobei eine Lichtquelle und ein Lichtempfänger der Messeinheit über die Messöffnung auf den darüber befindlichen Testbandabschnitt fokussiert sind.

Vorteilhafterweise ist die Bandführung durch ein Deckelteil nach außen abgedeckt, wobei der Auflagerahmen aus einem Durchbruch des Deckelteils herausragt.

Bevorzugt ist eine erfindungsgemäße Testbandeinheit als Bandkassette zum Einsetzen in ein Testgerät ausgebildet.

Im Folgenden wird die Erfindung anhand eines in der Zeichnung schematisch dargestellten Ausführungsbeispiels näher erläutert. Es zeigen
- Fig. 1: eine Bandkassette für Blutzuckertests in einer aufgebrochenen perspektivischen Darstellung;
- Fig. 2: eine Kopfpartie der Bandführung in der Bandkassette nach Fig. 1 in vergrößerter perspektivischer Ansicht; und
- Fig. 3: die Kopfpartie der Bandführung nach Fig. 2 in der Draufsicht;
- Fig. 4: ein Testsystem mit eingesetzter Bandkassette in einer teils geschnittenen, teils schematischen Darstellung.

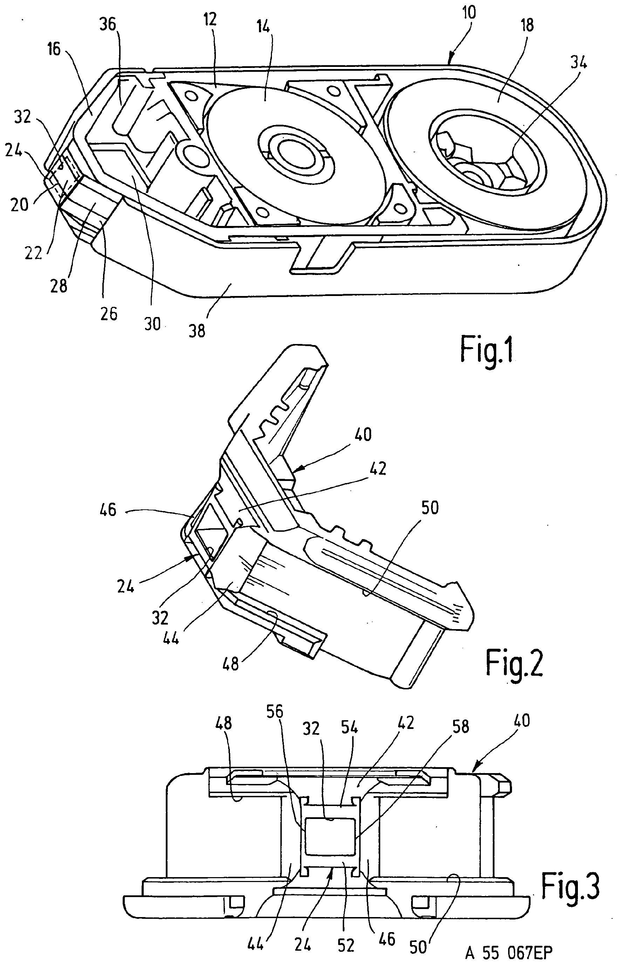

Die in Fig. 1 dargestellte Bandkassette 10 ermöglicht die Durchführung einer Vielzahl von Glucoseuntersuchungen an vor Ort vom Patienten selbst gewonnenen Blutproben. Zu diesem Zweck umfasst die Bandkassette 10 ein analytisches Testband 12, das von einer Vorratsspule 14 abziehbar und über eine Bandführung 16 auf eine Aufwickelspule 18 aufwickelbar ist, wobei ein Testbandabschnitt 20 an einer Messstelle 22 über einem ebenen Auflagerahmen 24 plan aufgespannt wird, um eine vorderseitige Applikation von Körperflüssigkeit (Blut bzw. Gewebeflüssigkeit) und eine präzise rückseitige reflektometrische Vermessung zu erlauben.

Das Testband 12 besteht aus einem lichtdurchlässigen Trägerband 26, auf dessen Vorderseite abschnittsweise Testfelder 28 etikettenartig aufgebracht sind. Diese enthalten Trockenchemikalien, die auf den Analyten (Glucose) in der applizierten Blutflüssigkeit ansprechen und bei rückseitiger Beleuchtung zu einer messbaren Veränderung der Lichtrückstrahlung führen. Beispielsweise besteht das Trägerband 26 aus einer 5 mm breiten und etwa 10 pm dicken Folie, auf die ein Nachweisfilm von 50 µm Dicke abschnittsweise vorderseitig aufgebracht ist.

Bei der Messung wird das Messlicht über eine von dem Auflagerahmen 24 umrandete Messöffnung 32 eingestrahlt und zurückgeworfen, ohne dass innerhalb des Öffnungsbereichs Optikelemente wie Linsen, Filter oder materiell ausgefüllte Fenster vorhanden sind (ggf. kann aber die Messöffnung durch eine Blende umrandet sein). Dies erlaubt eine definierte rückseitige Fokussierung bzw. Ausrichtung der optischen Messeinheit auf den Testbandabschnitt 20, der über der Messöffnung 32 plan freigelegt ist.

Um die Testfelder 28 sukzessive an die Messstelle 22 zu transportieren, ermöglicht ein in die Nabe 34 der Aufwickelspule 18 eingreifender Bandantrieb das Vorspulen des Testbands 12. Hierbei werden durch Reibung an der Vorratsspule 14 und im Bereich der Bandführung 16 (dort insbesondere an einer Durchzugdichtung 36) Rückhaltekräfte von etwa 2 N erzeugt, so dass das Testband 12 ausreichend unter Zug gesetzt wird, um eine ebene Auflage auf dem Auflagerahmen 24 zu gewährleisten.

Die Bandführung 16 ist durch ein Spritzgussteil aus Polypropylen gebildet, welches zugleich einen Trägerkörper für die Spulen 14, 18 bildet. Zur Abdeckung der Bandführung 16 nach außen ist ein Deckelteil 38 vorgesehen, welches an einer verjüngten Schmalseitenwand einen Durchbruch für eine einfach zugängliche Freilegung des Auflagerahmens 24 aufweist.

Wie am besten aus Fig. 2 ersichtlich, ist der Auflagerahmen 24 an einer Kopfpartie 40 der Bandführung 16 ausgebildet. Er wird hier durch die ebene Deckfläche eines pyramidenstumpfförmig verjüngten Vorsprungs 42 gebildet, so dass eine hygienische Applikation auch kleiner Blutmengen erleichtert wird. In Bandlängsrichtung gesehen schließen sich somit Umlenkschrägen 44, 46 an den Auflagerahmen 24 an, um das Testband 12 an den einander gegenüberliegenden Längsseitenwänden der Kassette 10 aus- und einzuleiten zu können. In diesem Bereich sind auch Seitenbegrenzungen 48, 50 vorgesehen, die das Testband 12 gegen Querauslenkung sichern, so dass ein seitliches Verrutschen auf dem Auflagerahmen 24 vermieden wird.

Wie auch aus Fig. 3 zu erkennen, besitzt der Auflagerahmen 24 zwei längs des darüber geführten Testbands 16 verlaufende Rahmenschenkel 52, 54 und zwei rechtwinklig dazu sich erstreckende Rahmenschenkel 56, 58. Die längslaufenden Rahmenschenkel 52, 54 sind streifenförmig abgeflacht und besitzen einen Außenrandabstand voneinander, der kleiner als die Breite des Testbands 16 ist. Auf diese Weise wird durch das seitlich überstehende Testband 16 verhindert, dass Blut bei der Applikation auf den Vorsprung 42 gelangt. Die quer verlaufenden Rahmenschenkel 56, 58 sind an den Umlenkkanten 43 für das Testband 16 zweckmäßig über einen Radius von etwa 0,3 mm abgerundet und in ihrer Länge so bemessen, dass das Testband 16 über seine gesamte Breite gestützt wird. Bei einer Bandbreite von 5 mm besitzen die Rahmenschenkel 56, 58 somit ebenfalls eine Länge von 5 mm, während die Messöffnung 32 eine Länge von 3 mm und eine Breite von 2 mm aufweist. Durch diese Gestaltung des Auflagerahmens 24 wird neben einer hygienischen Handhabung auch erreicht, dass sich der aufgespannte Testbandabschnitt 20 an der Messstelle nicht einwölbt.

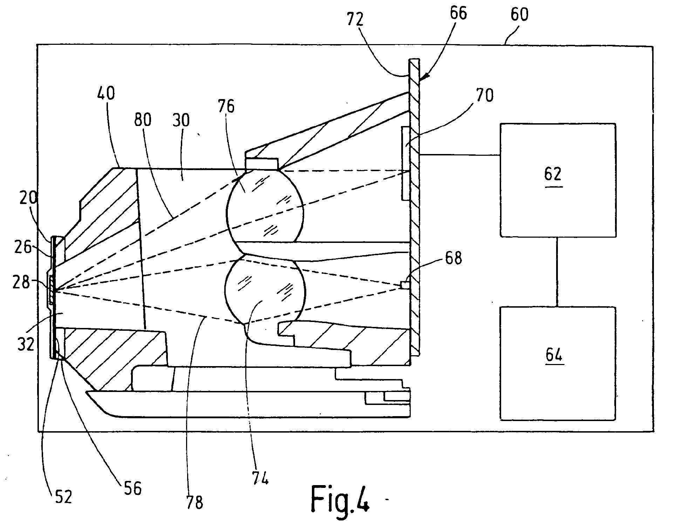

Zur Vereinfachung der Handhabung wird die Kassette 10 als Verbrauchsmaterial in ein Handgerät 60 eingesetzt, wie es in Fig. 4 als Blockschaltbild symbolisiert ist. Das Gerät 60 weist eine Steuer- und Auswerteeinheit 62, einen auf die Nabe 34 der Aufwickelspule 18 einwirkenden Bandantrieb 64 sowie einen in die kassettenseitige Messkammer 30 eingreifenden optischen Messkopf 66 auf.

Der Messkopf 66 und die Kopfpartie 40 der Kassette 10 sind in Fig. 4 in einem Schnitt in Querrichtung des Testbands dargestellt. Der Messkopf 66 umfasst eine Lichtquelle 68 und einen Lichtempfänger 70 auf eine Platine 72. Die Lichtquelle 68 und der Lichtempfänger 70 sind über messkopfseitige Linsen 74, 76 durch das transparente Trägerband 26 hindurch auf das Testfeld 28 fokussiert. Dabei treten sende- und empfangsseitige Lichtbündel 78, 80 durch die Messkammer 30 und insbesondere die Messöffnung 32 hindurch, ohne dass eine weitere Wechselwirkung mit optischen Bauelementen stattfindet. Auf diese Weise ist ein reflektionsphotometrischer Nachweis in einer definierter Messebene des Testbands möglich, wobei der Strahlengang nicht durch Bauelemente der Kassette 10 negativ beeinflusst wird.

## Patentansprüche

1. Testsystem mit einer reflektometrisch arbeitenden Messeinheit, einem Bandantrieb und einer als Verbrauchsmaterial einsetzbaren Testbandeinheit, insbesondere für Blutzuckertests, wobei die Testbandeinheit ein analytisches Testband (12), eine Vorratsspule (14) zum Abspulen von unverbrauchtem Testband (12) sowie eine Aufwickelspule (18) zum Aufwickeln von verbrauchtem Testband (12) und eine Bandführung (16) zur Exposition eines Testbandabschnitts (20) an einer Messstelle (22) für eine Applikation von Körperflüssigkeit auf die Bandvorderseite aufweist, **dadurch gekennzeichnet, dass** die Bandführung (16) einen ebenen Auflagerahmen (24) aufweist, welcher den Testbandabschnitt (20) an der Messstelle (22) plan aufspannt und dabei mit seinen Rahmenschenkeln (52,54,56,58) eine von Optikelementen freigehaltene lichte Messöffnung (32) für eine optische Messung umlaufend begrenzt, wobei an dem auf dem Auflagerahmen (24) aufliegenden Testbandabschnitt (20) mittels der Messeinheit eine reflektometrische Messung von der Bandrückseite her erfolgt und wobei der Auflagerahmen (24) zwei in Längsrichtung des Testbands (12) nebeneinander verlaufende Rahmenschenkel (52,54) aufweist, deren Außenrandabstand kleiner als die Breite des Testbands (12) ist.

2. Testsystem nach Anspruch 1, **dadurch gekennzeichnet, dass** die Rahmenschenkel (52,54,56,58) des Auflagerahmens (24) die Messöffnung (32) rechteckförmig begrenzen.

3. Testsystem nach Anspruch 1 oder 2, **dadurch gekennzeichnet, dass** die Breite der Messöffnung (32) in Bandquerrichtung gesehen das 0,3 bis 0,6-fache der Breite des Testbands (12) beträgt.

4. Testsystem nach einem der Ansprüche 1 bis 3, **dadurch gekennzeichnet, dass** Länge der Messöffnung (32) in Bandlängsrichtung gesehen das 1,0 bis 2,0-fache der Breite der Messöffnung (32) beträgt.

5. Testsystem nach einem der Ansprüche 1 bis 4, **dadurch gekennzeichnet, dass** Auflagerahmen (24) zwei in Querrichtung des Testbands (12) parallel verlaufende Rahmenschenkel (56,58) aufweist, deren Länge zumindest der Breite des Testbands (12) entspricht.

6. Testsystem nach einem der Ansprüche 1 bis 5, **dadurch gekennzeichnet, dass** die in Längsrichtung des Testbands (12) verlaufenden Rahmenschenkel (52, 54) des Auflagerahmens (24) streifenförmig abgeflacht sind.

7. Testsystem nach einem der Ansprüche 1 bis 6, **dadurch gekennzeichnet, dass** die quer zu dem Testband (12) verlaufenden Rahmenschenkel (56,58) des Auflagerahmens (24) an einer Umlenkkante für das Testband (12) vorzugsweise über einen Radius von 0,2 bis 0,5 mm abgerundet sind.

8. Testsystem nach einem der Ansprüche 1 bis 7, **dadurch gekennzeichnet, dass** der Auflagerahmen (24) durch die ebene Deckfläche eines vorzugsweise pyramidenstumpfförmig verjüngten Vorsprungs (42) der Bandführung (16) gebildet ist.

9. Testsystem nach einem der Ansprüche 1 bis 8, **dadurch gekennzeichnet, dass** die Bandführung (16) in Bandlängsrichtung an dem Auflagerahmen (24) anschließende Umlenkschrägen (44,46) aufweist, wobei die Umlenkschrägen (44,46) einen spitzen Winkel vorzugsweise im Bereich von 70° bis 85° einschließen.

10. Testsystem nach einem der Ansprüche 1 bis 9, **dadurch gekennzeichnet, dass** die Bandführung (16) außerhalb des Bereichs des Auflagerahmens (24) angeordnete Seitenbegrenzungen (48,50) aufweist, welche das Testband (12) gegen Querauslenkung sichern.

11. Testsystem nach einem der Ansprüche 1 bis 10, **dadurch gekennzeichnet, dass** das Testband (12) durch Antrieb der Aufwickelspule (18) von der Vorratsspule (14) abspulbar ist, wobei das Testband (12) durch Rückhaltekräfte vorzugsweise von mehr als 1 N unter Zug gehalten ist.

12. Testsystem nach einem der Ansprüche 1 bis 11, **dadurch gekennzeichnet, dass** der Auflagerahmen (24) an einem Formteil (40) einstückig angeformt ist.

13. Testsystem nach Anspruch 12, **dadurch gekennzeichnet, dass** das Formteil (40) vorzugsweise aus schwarz eingefärbtem Polypropylen als Spritzgussteil ausgebildet ist.

14. Testsystem nach einem der Ansprüche 1 bis 13, **dadurch gekennzeichnet, dass** die Körperflüssigkeit auf die freie Bandvorderseite des auf dem Auflagerahmen (24) abgestützten Testbandabschnitts (20) applizierbar ist.

15. Testsystem nach einem der Ansprüche 1 bis 14, **dadurch gekennzeichnet, dass** eine von der Bandführung (16) begrenzte Messkammer (36) für den Einsatz einer Messeinheit ausgebildet ist, wobei eine Lichtquelle und ein Lichtempfänger der Messeinheit über die Messöffnung (32) rückseitig auf den darüber befindlichen Testbandabschnitt (20) fokussiert sind.

16. Testsystem nach einem der Ansprüche 1 bis 15, **dadurch gekennzeichnet, dass** die Bandführung (16) durch ein Deckelteil (38) nach außen abgedeckt ist, wobei der Auflagerahmen (24) aus einem Durchbruch des Deckelteils (38) herausragt.

17. Testsystem nach einem der Ansprüche 1 bis 16, **dadurch gekennzeichnet, dass** die Testbandeinheit als Bandkassette (10) zum Einsetzen in ein Testgerät ausgebildet ist.

18. Testsystem nach einem der Ansprüche 1 bis 17, **dadurch gekennzeichnet, dass** der Bandantrieb die Aufwickelspule antreibt, wobei das Testband (12) durch Rückhaltekräfte vorzugsweise von mehr als 1 N unter Zug gehalten ist.

19. Testsystem nach einem der Ansprüche 1 bis 18, **dadurch gekennzeichnet, dass** die Messeinheit den exponierten Testbandabschnitt (20) über mindestens ein Lichtbündel optisch abtastet, wobei das Lichtbündel im Bereich der Messöffnung frei von einer Wechselwirkung mit materiellen Optikelementen wie Linsen, Filter oder Fenster hindurchtritt.

20. Testsystem nach einem der Ansprüche 1 bis 19, **dadurch gekennzeichnet, dass** eine Lichtquelle und ein Lichtempfänger der Messeinheit durch die Messöffnung (32) hindurch rückseitig auf den Testbandabschnitt (20) ausgerichtet sind.

## Claims

1. Test system comprising a reflectometric measuring unit, a tape drive and a test tape unit that can be used as a consumable material, in particular for blood sugar tests, wherein the test tape unit comprises an analytical test tape (12), a feed spool (14) for unwinding unused test tape (12) and a take-up spool (18) for winding used test tape (12) and a tape guide (16) to expose a section of test tape (20) at a measuring site (22) for an application of body fluid onto the front side of the tape, **characterized in that** the tape guide (16) has a planar support frame (24) which holds the test tape section (20) flat at the measuring site (22), whereby the frame legs (52, 54, 56, 58) of the support frame circumferentially border a clear measuring opening (32) which is kept free from optical elements for an optical measurement, wherein a reflectometric measuring from the rear side of the tape is effected on the test tape section (20) supported on the support frame (24) and wherein the support frame (24) has two parallel frame legs (52,54) extending in the longitudinal direction of the test tape (12) the distance between the outer edges of which is less than the width of the test tape (12).

2. Test system according to claim 1, **characterized in that** the frame legs (52, 54, 56, 58) of the support frame (24) border the measuring opening (32) in a rectangular shape.

3. Test system according to claim 1 or 2, **characterized in that** the width of the measuring opening (32) at right angles to the tape direction is 0.3 to 0.6-times the width of the test tape (12).

4. Test system according to one of the claims 1 to 3, **characterized in that** the length of the measuring opening in the longitudinal tape direction is 1.0 to 2.0-times the width of the measuring opening (32).

5. Test syste according to one of the claims 1 to 4, **characterized in that** the support frame (24) has two parallel frame legs (56, 58) at right angles to the direction of the test tape (12), the length of which corresponds to at least the width of the test tape (12).

6. Test system according to one of the claims 1 to 5, **characterized in that** the frame legs (52, 54) of the support frame (24) extending in the longitudinal direction of the test tape (12) are flattened in a strip shape.

7. Test system according to one of the claims 1 to 6, **characterized in that** the frame legs (56, 58) that are at right angles to the test tape (12) are rounded off at a deflecting edge for the test tape (12) preferably over a radius of 0.2 to 0.5 mm.

8. Test system according to one of the claims 1 to 7, **characterized in that** the support frame (24) is formed by the flat top surface of a preferably truncated pyramid-shaped, tapered projection (42) of the tape guide (16).

9. Test system according to one of the claims 1 to 8, **characterized in that** the tape guide (16) has deflecting bevels (44, 46) adjoining the support frame (24) in the longitudinal direction of the tape, wherein the deflecting bevels (44, 46) enclose an acute angle preferably in the range of 70° to 85°.

10. Test system according to one of the claims 1 to 9, **characterized in that** the tape guide (16) has side boundaries (48, 50) which are arranged outside of the area of the support frame (24) which secure the test tape (12) against lateral deflection.

11. Test system according to one of the claims 1 to 10, **characterized in that** test tape (12) can be unwound from the feed spool (14) by driving the take-up spool (18), wherein the test tape (12) is held under tension by return forces preferably of more than 1 N.

12. Test system according to one of the claims 1 to 11, **characterized in that** the support frame (24) is moulded as one piece onto a moulded part (40).

13. Test system according to claim 12, **characterized in that** the moulded piece (40) is made preferably from black coloured polypropylene as an injection-moulded part.

14. Test system according to one of the claims 1 to 13, **characterized in that** the body fluid can be applied to the free front side of the test tape section (20) which is supported by the support frame (24).

15. Test system according to one of the claims 1 to 14, **characterized in that** a measuring chamber (36) delimited by the tape guide (16) is designed to be used with a measuring unit, wherein a light source and a light receiver of the measuring unit are focussed through the measuring opening (32) onto the test tape section (20) that is located above it.

16. Test system according to one of the claims 1 to 15, **characterized in that** the tape guide (16) is covered from the outside by a cover part (38), wherein the support frame (24) protrudes from an opening of the cover part (38).

17. Test system according to one of the claims 1 to 16, **characterized in that** the test tape unit is designed as a tape cassette (10) for insertion into a test device.

18. Test system according to one of the claims 1 to 17, **characterized in that** the tape drive drives the take-up spool, wherein the test tape (12) is held under tension by retention forces preferably of more than 1 N.

19. Test system according to one of the claims 1 to 18, **characterized in that** the measuring unit optically scans the exposed section of test tape (20) by means of at least one light beam whereby the light beam passes through the area of the measuring opening free of any interaction with material optical elements such as lenses, filters or windows.

20. Test system according to one of the claims 1 to 19, **characterized in that** a light source and a light receiver of the measuring unit are aligned through the measuring opening (32)onto the section of test tape (20) on the rear side.

## Revendications

1. Système de test comportant une unité de mesure fonctionnant par réflectométrie, un entraînement à courroie et une unité de bande de test pouvant être utilisée comme consommable, en particulier pour les tests de glycémie, l'unité de bande de test présentant une bande de test analytique (12), une bobine d'alimentation (14) pour dérouler une bande de test inutilisée (12) et une bobine d'enroulement (18) pour enrouler la bande de test épuisée (12), et un guide de bande (16) pour exposer une section de bande de test (20) en un point de mesure (22) pour l'application de fluide corporel sur la face avant de la bande, **caractérisé en ce que** le guide de bande (16) présente un cadre de support plan (24), lequel couvre la section de bande de test (20) au niveau du point de mesure (22) à plat, et, par ses branches de cadre (52, 54, 56, 58), délimite ainsi circonférentiellement une ouverture de mesure (32) dégagée, tenue exempte d'éléments optiques pour une mesure optique, la section de bande de test (20) reposant sur le cadre de support (24) étant soumise à une mesure réflectométrique au moyen de l'unité de mesure, à partir de l'arrière de la bande, et le cadre de support (24) présentant deux branches de cadre (52, 54) juxtaposées dans la direction longitudinale de la bande de test (12), la distance extérieure séparant les branches étant inférieure à la largeur de la bande de test (12).

2. Système de test selon la revendication 1, **caractérisé en ce que** les branches de cadre (52, 54, 56, 58) du cadre de support (24) délimitent l'ouverture de mesure (32) de manière rectangulaire.

3. Système de test selon la revendication 1 ou 2, **caractérisé en ce que** la largeur de l'ouverture de mesure (32), vue dans la direction transversale de la bande, est de 0,3 à 0,6 fois la largeur de la bande de test (12).

4. Système de test selon l'une des revendications 1 à 3, **caractérisé en ce que** la longueur de l'ouverture de mesure (32), vue dans la direction longitudinale de la bande, est de 1,0 à 2,0 fois la largeur de l'ouverture de mesure (32).

5. Système de test selon l'une des revendications 1 à 4, **caractérisé en ce que** le cadre de support (24) présente deux branches de cadre (56, 58) s'étendant parallèlement dans la direction transversale de la bande de test (12), la longueur des branches correspondant au moins à la largeur de la bande de test (12).

6. Système de test selon l'une des revendications 1 à 5, **caractérisé en ce que** les branches de cadre (52, 54) du cadre de support (24) s'étendant dans la direction longitudinale de la bande de test (12) sont aplaties en forme de bande.

7. Système de test selon l'une des revendications 1 à 6, **caractérisé en ce que** les branches de cadre (56, 58) du cadre de support (24) s'étendant transversalement à la bande (12) sont arrondies au niveau d'un bord de déviation pour la bande de test (12), de préférence sur un rayon de 0,2 à 0,5 mm.

8. Système de test selon l'une des revendications 1 à 7, **caractérisé en ce que** le cadre de support (24) est formé par la surface de recouvrement plane d'une saillie (42) du guide de bande (16), laquelle saillie est de préférence conique en forme de pyramide tronquée.

9. Système de test selon l'une des revendications 1 à 8, **caractérisé en ce que** le guide de bande (16) présente des pentes de flexion (44, 46) adjacentes au cadre de support (24) dans la direction longitudinale de la bande, les pentes de flexion (44, 46) entourant un angle aigu, de préférence dans la plage de 70° à 85°.

10. Système de test selon l'une des revendications 1 à 9, **caractérisé en ce que** le guide de bande (16) présente des limites latérales (48, 50) disposées à l'extérieur de la zone du cadre de support (24), lesquelles protègent la bande de test (12) contre une déformation transversale.

11. Système de test selon l'une des revendications 1 à 10, **caractérisé en ce que** la bande de test (12) peut être déroulée de la bobine d'alimentation (14) par entraînement de la bobine d'enroulement (18), la bande de test (12) étant maintenue sous tension par des forces de retenue, de préférence supérieures à 1 N.

12. Système de test selon l'une des revendications 1 à 11, **caractérisé en ce que** le cadre de support (24) est formé d'un seul tenant sur une pièce moulée (40).

13. Système de test selon la revendication 12, **caractérisé en ce que** la pièce moulée (40) est de préférence réalisée comme pièce moulée par injection, en polypropylène teint en noir.

14. Système de test selon l'une des revendications 1 à 13, **caractérisé en ce que** le fluide corporel peut être appliqué sur le côté avant de la bande libre de la section de bande de test (20) reposant sur le cadre de support (24).

15. Système de test selon l'une des revendications 1 à 14, **caractérisé en ce que** l'une des chambres de mesure (36) délimitée par le guide de bande (16) est conçue pour l'utilisation d'une unité de mesure, une source de lumière et un récepteur de lumière de l'unité de mesure étant focalisés à travers l'ouverture de mesure (32) à l'arrière sur la section de bande de test superposée (20).

16. Système de test selon l'une des revendications 1 à 15, **caractérisé en ce que** le guide de bande (16) est recouvert vers l'extérieur par une partie de couvercle (38), le cadre de support (24) faisant saillie depuis une ouverture de la partie de couvercle (38).

17. Système de test selon l'une des revendications 1 à 16, **caractérisé en ce que** l'unité de bande de test est conçu comme une cassette à bande (10) à insérer dans un appareil de test.

18. Système de test selon l'une des revendications 1 à 17, **caractérisé en ce que** l'entraînement de bande entraîne la bobine d'enroulement, la bande de test (12) étant maintenue sous tension par des forces de retenue de préférence supérieures à 1 N.

19. Système de test selon l'une des revendications 1 à 18, **caractérisé en ce que** l'unité de mesure balaie optiquement la section de bande de test exposée (20) par au moins un faisceau de lumière, le faisceau de lumière passant dans la zone de l'ouverture de mesure sans interaction avec des éléments optiques physiques tels que des lentilles, des filtres ou des fenêtres.

20. Système de test selon l'une des revendications 1 à 19, **caractérisé en ce qu'une** source de lumière et un récepteur de lumière de l'unité de mesure sont alignés à travers l'ouverture de mesure (32) à l'arrière sur la section de bande de test (20).
